# EUROPEAN PATENT APPLICATION

(11) **EP 4 591 875 A1**
(43) Date of publication of application: **30.07.2025**
(21) Application number: 23867595.3
(22) Date of filing: 21.09.2023
(51) Int. Cl.: A61K 38/26, C07K 14/605, A61P 3/10

(54) **DUAL GLP-1 AND GIP RECEPTOR AGONIST PHARMACEUTICAL COMPOSITION AND USE THEREOF**

(30) Priority: 23.09.2022 CN 202211167785
(71) Applicant: Brightgene Pte. Ltd., Singapore 139949 (SG)
(72) Inventor: LI, Huifang, Suzhou, Jiangsu 215123 (CN); FENG, Xin, Suzhou, Jiangsu 215123 (CN); ZHANG, Bo, Suzhou, Jiangsu 215123 (CN); ZHENG, Fangfang, Suzhou, Jiangsu 215123 (CN); LI, Jiahan, Suzhou, Jiangsu 215123 (CN); SHENG, Panpan, Suzhou, Jiangsu 215123 (CN)
(74) Representative: Bittner, Bernhard
(86) International application number: PCT/CN2023/120363
(87) International publication number: WO 2024/061310

(57) **Abstract**

The present invention provides a dual GLP-1 and GIP receptor agonist pharmaceutical composition, comprising a dual GLP-1 and GIP receptor agonist, a stabilizer and a buffer salt. The present invention also provides use of the pharmaceutical composition in the preparation of a drug for preventing and/or treating metabolic disorder related diseases.

## Description

The present disclosure claims the right of priority for the following patent applications: a Chinese patent application with the application number 202211167785.2, submitted to the China National Intellectual Property Administration on September 23, 2022; and the entire contents of the above-mentioned patent application are incorporated in the present disclosure by reference.

### Technical Field

The present disclosure belongs to the technical field of medicine, and specifically, the present disclosure relates to a dual GLP-1 and GIP receptor agonist pharmaceutical composition and use thereof.

### Background Art

Glucose-dependent insulinotropic peptide (GIP) is a gastrointestinal regulatory peptide having 42 amino acids and plays a physiological role in glucose homeostasis by stimulating insulin secretion from pancreatic β cells in the presence of glucose and protecting pancreatic β cells. Glucagon-like peptide-1 (GLP-1) is a peptide having 37 amino acids and stimulates insulin secretion, protects pancreatic β cells, and inhibits glucagon secretion, gastric emptying, and food intake, thereby leading to body weight loss. GIP and GLP-1 are secreted by K cells and L cells of the small intestinal endothelium, respectively, and are referred to as incretins, and incretin receptor signaling plays a key physiologically relevant role in glucose homeostasis.

GIP and GLP-1 produce corresponding physiological effects by binding to specific receptors thereof, and bind to GIP (GIPR) receptor and GLP-1 receptor (GLP-1R), respectively. Studies have shown that co-activation of GLP-1R/GIPR can exert a synergistic hypoglycemic effect, and a dual GIP and GLP-1 receptor agonist can produce more excellent hypoglycemic effect and stimulation of insulin secretion. Therefore, the development of a dual GLP-1 and GIP receptor agonist which is structurally novel and its pharmaceutical composition, which have good therapeutic efficacy, has been one of the top research points in the field of treatment of metabolic diseases such as diabetes or obesity.

At present, polypeptide-based drugs are mostly preserved and used in liquid preparations, the polypeptide active ingredients are susceptible to the external environment, thereby undergoing chemical changes such as hydrolysis and oxidation, and the resulting impurities can seriously affect the effectiveness and safety of the drugs.

Dual GLP-1 and GIP receptor agonists are generally linear peptides. Furthermore, since the pH of the preparations needs to be close to the physiological pH of the human body, the pH of the preparations is generally controlled to about 7 to 8, and the polypeptide-based drugs are extremely susceptible to degradation by microorganisms at this pH. In general, the addition of antimicrobial agents (such as phenol) to prescriptions is considered to improve the product stability. For example, the commercially available hypoglycemic drug preparation, semaglutide (OZEMPIC) has 8.25 mg of phenol added as an antimicrobial agent; exenatide has 2.64 mg m-cresol added as an antimicrobial agent; liraglutide (VICTOZA) has 16.5 mg phenol added as an antimicrobial agent, and lixisenatide (ADLYXIN) has 8.1 mg m-cresol added as an antimicrobial agent. However, since the injection may directly enter the circulation in the human body, the use of antimicrobial agents may increase the risk of drug safety. The national regulatory agencies have also issued several documents indicating that the use of preservatives should be reduced as much as possible when screening prescriptions of insulin products. Therefore, the development of antimicrobial agent-free prescriptions of polypeptide-based hypoglycemic drugs is also a technical challenge in the art, and the development of preservative/antimicrobial agent-free dual GLP-1 and GIP receptor agonist preparations will have good application prospects.

In addition, polypeptide-based drugs are formed by connecting multiple amino acids with each other through the formation of amide bonds via condensation reactions, and have a large number of electron-rich and electron-withdrawing groups; therefore, polypeptide-based drugs are very prone to intermolecular attraction when prepared into pharmaceutical preparations, leading to the polymerization of drug molecules. Polypeptide-based drugs are not effective once they are polymerized. Furthermore, drug polymerization may cause quality problems such as turbidity or discoloration of injections. Therefore, solving the problem of drug polymerization in the preparation of polypeptide-based drugs into preparations has also always been a technical difficulty in the art.

In summary, the development of formulas of dual GLP-1 and GIP receptor agonist pharmaceutical compositions that are suitable for long-term preservation and are stable in their efficacy is a problem urgently to be solved in the art.

### Summary of the Invention

### Problems to be solved by the invention

In order to develop a dual GLP-1 and GIP receptor agonist that is structurally novel and is capable of effectively treating obesity, diabetes and their complications, a dual GLP-1 and GIP receptor agonist is provided by the applicant of the present application in a patent application with the application number 202210294984.3, entitled "Dual GIP and GLP-1 receptor agonists, pharmaceutical compositions and use". The dual receptor agonist is a glucose-dependent dual GLP-1 and GIP (as incretin polypeptides) receptor agonist developed independently by the applicant in the present application, which is clinically intended for type 2 diabetes as an indication. The drug is a 40-amino acid linear peptide that has an agonistic effect on GLP-1R and GIPR. Preclinical studies have shown that the dual receptor agonist has a strong agonistic effect on both GLP-1 receptor and GIP receptor, with a 2- to 3-fold greater activity compared to Tirzepatide; the side chain is composed of a fatty acid chain linked to double AEEEA chains, and the main function of the side chain is to bind to albumin (albumin having basic residues) in the blood, thereby extending the half-life and achieving long-lasting effects. The results of pre-clinical pharmacokinetic studies indicated that the binding rates of dual receptor agonist to plasma proteins from all species were all over 99.4%, and half-lives in rats and cynomolgus monkeys were about 14h and 40h, respectively.

For the above dual GLP-1 and GIP receptor agonist, no pharmaceutical preparation suitable for long-term storage and keeping stable efficacy has been developed. In this regard, the present disclosure is intended to provide a dual GLP-1 and GIP receptor agonist pharmaceutical composition and use thereof, and the dual GLP-1 and GIP receptor agonist are kept stable in a liquid preparation by addition of auxiliary materials such as stabilizers and buffer salts, thereby effectively preventing the physical and chemical degradation of the dual receptor agonist.

### Solutions to solve problems

A first aspect of the present disclosure provides a pharmaceutical composition, comprising a dual GLP-1 and GIP receptor agonist, a stabilizer, and a buffer salt; wherein the dual GLP-1 and GIP receptor agonist is a compound represented by formula I or a pharmaceutically acceptable salt, ester, solvate, optical isomer, tautomer, isotopically labeled compound or prodrug thereof:

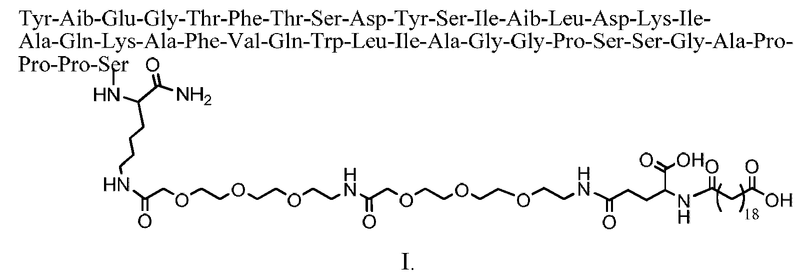

Further, the pharmaceutical composition is a liquid preparation; preferably, the pharmaceutical composition is an injection.

Further, the concentration of the dual GLP-1 and GIP receptor agonist in the pharmaceutical composition is 2-36 mg/mL, preferably 5-30 mg/mL.

Further, in the pharmaceutical composition, the stabilizer is a polyol; preferably, the stabilizer is propylene glycol, mannitol or glycerin; and more preferably, the stabilizer is propylene glycol.

Still further, the concentration of the stabilizer in the pharmaceutical composition is 10-20 mg/mL.

Further, in the pharmaceutical composition, the buffer salt is selected from a hydrogen phosphate, a hydrogen phosphate hydrate, a citrate, or a citrate hydrate; preferably, the buffer salt is disodium hydrogen phosphate dodecahydrate or sodium citrate dihydrate; and more preferably, the buffer salt is disodium hydrogen phosphate dodecahydrate.

Still further, the concentration of the buffer salt in the pharmaceutical composition is 7-23 mM.

Further, the pH of the pharmaceutical composition is 6.5-8.5; preferably, the pH of the pharmaceutical composition is 7.0-8.4; preferably, the pH of the pharmaceutical composition is 7.2-8.2; and more preferably, the pH of the pharmaceutical composition is 7.2-7.6.

Still further, the pharmaceutical composition further comprises a pH regulator and water; the pH regulator is hydrochloric acid and/or sodium hydroxide; the pH of the pharmaceutical composition is adjusted by the pH regulator; and preferably, the water is purified water and/or water for injection.

Further, the pharmaceutical composition does not comprise an antimicrobial agent.

A second aspect of the present invention provides a pharmaceutical composition, comprising a dual GLP-1 and GIP receptor agonist represented by formula I, a stabilizer which is propylene glycol, and a buffer salt which is disodium hydrogen phosphate dodecahydrate, wherein the pH of the pharmaceutical composition is 7.0-8.4; moreover, in the pharmaceutical composition, the concentration of the dual GLP-1 and GIP receptor agonist represented by formula I is 5-30 mg/mL, the concentration of the propylene glycol is 10-20 mg/mL, and the concentration of the disodium hydrogen phosphate dodecahydrate is 7-23 mM; 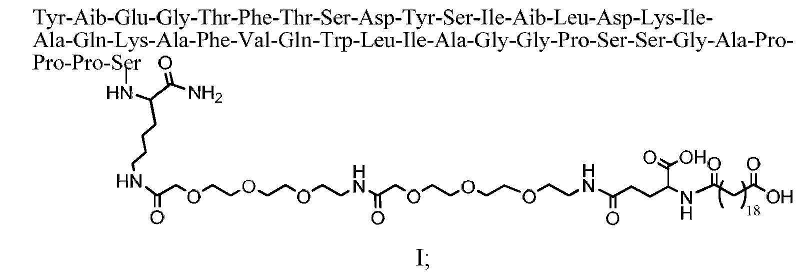 and the pharmaceutical composition is an injection.

Further, the pH of the pharmaceutical composition is 7.2-8.2, and the concentration of the disodium hydrogen phosphate dodecahydrate in the pharmaceutical composition is 10-20 mM.

Further, the pH of the pharmaceutical composition is 7.2-7.6; and in the pharmaceutical composition, the concentration of the dual GLP-1 and GIP receptor agonist represented by formula I is 5-25 mg/mL, the concentration of the propylene glycol is 14 mg/mL, and the concentration of the disodium hydrogen phosphate dodecahydrate is 10 mM.

Further, the pharmaceutical composition further comprises a pH regulator and water; preferably, the pH regulator is hydrochloric acid and/or sodium hydroxide; and preferably, the water is purified water and/or water for injection.

A third aspect of the present disclosure provides a method of preparing the pharmaceutical composition of the first or second aspect, comprising the following steps: formulating the stabilizer, the buffer salt and the dual GLP-1 and GIP receptor agonist into a solution, and adjusting the pH of the solution to a target value to obtain the pharmaceutical composition.

Further, the preparation method includes the following steps: dissolving the stabilizer and the buffer salt in a solvent, adding the dual GLP-1 and GIP receptor agonist to formulate into a solution, adding the pH regulator to make the dual GLP-1 and GIP receptor agonist dissolved completely, and adjusting the pH of the solution to a target value using the pH regulator to obtain the pharmaceutical composition.

A fourth aspect of the present disclosure provides the use of the pharmaceutical composition of the first or second aspect in the preparation of a drug for preventing and/or treating metabolic disorder related diseases; preferably, the metabolic disorder related diseases are diabetes, diabetes complications, obesity, or obesity complications.

### Effects of the Invention

In the present disclosure, by addition of an auxiliary material such as a stabilizer and a buffer salt in the pharmaceutical composition provided, the dual GLP-1 and GIP receptor agonist as the pharmaceutical active ingredient maintains good stability in a liquid preparation, and meanwhile, quality problems are avoided, such as efficacy loss of drugs due to the polymerization between dual GLP-1 and GIP receptor agonists as polypeptide-based drugs. Ultimately, the long-term preservation of the pharmaceutical composition is achieved, and the present disclosure exhibits an important application prospect in the prevention and treatment of metabolic disorder related diseases such as diabetes or diabetes complications, and obesity or obesity complications.

In the present disclosure, a pH regulator is utilized to control the pH of the pharmaceutical composition within a particular range, which is more beneficial for making the dual GLP-1 and GIP receptor agonist dissolved completely during the preparation of the pharmaceutical composition, and ensures the stability and prevents degradation of the dual GLP-1 and GIP receptor agonist as the pharmaceutical active ingredient during storage of the composition.

In the present disclosure, by selecting a specific stabilizer, the solubility of the dual GLP-1 and GIP receptor agonist (such as the compound of formula I) is greatly improved while the dual receptor agonist is allowed to have a complete structure and complete biological activity during storage.

In the present disclosure, selecting a particular buffer salt is able to ensure that the pH of the pharmaceutical composition is stable during storage without affecting the quality and stability of the pharmaceutical active ingredient.

The method for preparing the pharmaceutical composition provided in the present disclosure is simple to operate, low in production cost, and beneficial for scale-up production, and assist in a wide range of application of the pharmaceutical preparation.

The pharmaceutical composition provided in the present disclosure are effective in preventing and/or treating metabolic disorder related diseases such as diabetes, diabetes complications, obesity or obesity complications, and can be used in a convenient manner.

### Brief Description of the Drawings

FIG. 1 shows chromatograms for verifying the specificity of a multimer detection method.
FIG. 2 shows chromatograms of multimer detection of the injection of the compound of formula I.

### Detailed Description of Embodiments

### <Definitions>

Unless stated to the contrary, the terms used in the present disclosure have the following meanings.

In the claims and/or specification of the present invention, the words "a", "an" or "the" can refer to "one", but can also refer to "one or more", "at least one" and "one or more than one".

As used in the claims and the description, the words "comprising", "having", "including" or "containing" refer to an inclusive and open-ended expression, which does not exclude additional, unquoted elements or method steps. Moreover, "comprising", "having", "including" or "containing" may also refer to a closed expression, which excludes additional, unquoted elements or method steps.

In the present disclosure, the term "about" means: a value comprising the standard deviation in the error caused by the device or method used to determine the value.

The term "agonist" used in the present disclosure refers to a substance (ligand) that activates a signaling pathway through the targeted receptor type. For example, if a GLP-1 receptor is a target receptor, the agonist has the activation activity of GLP-1 receptor, and is such as a GLP-1 polypeptide or an analog thereof.

In the present disclosure, the term "treating" or "treatment" refers to: after suffering from a disease, alleviation of symptoms of the disease when a subject is exposed to (e.g., dosed with) the pharmaceutical composition of the present disclosure, as compared to when not exposed, and it does not mean that the symptoms of the disease must be completely suppressed. Suffering from a disease means that: the body has symptoms of the disease.

In the present disclosure, the term "preventing" or "prevention" refers to: before suffering from a disease, alleviation of symptoms after suffering from the disease when a subject is exposed to (e.g., dosed with) the pharmaceutical composition of the present disclosure, as compared to when not exposed, and it does not mean that suffering from the disease must be completely suppressed.

In the present disclosure, the term "individual", "patient" or "subject" include mammals. Mammals include, but are not limited to, domesticated animals (e.g., cows, sheep, cats, dogs, and horses), primates (e.g., human, and non-human primates such as monkeys), rabbits, and rodents (e.g., mice and rats).

In the present disclosure, the term "therapeutically effective amount" refers to an amount effective to achieve the desired therapeutic result at a dose and for periods of time desired. The therapeutically effective amount of the compound or pharmaceutical composition of the present disclosure can vary depending on factors such as disease state, age, sex and weight of an individual, and the ability of an immune adjuvant or the pharmaceutical composition to activate a desired response in an individual.

In the present disclosure, the term "pharmaceutically acceptable salt" refers to a salt prepared from the compound of the present disclosure with a relatively non-toxic acid or base. When the compound of the present disclosure contains a relatively acidic functional group (such as carboxy or sulfonic acid group), a base addition salt can be obtained by contacting the free form of the compound with a sufficient amount of base, either in a pure solution or a suitable inert solvent. Non-limiting examples of pharmaceutically acceptable base addition salts include, but are not limited to, sodium salts, potassium salts, ammonium salts, calcium salts, magnesium salts, organic amine salts, or similar salts. When the compound of the present disclosure contains a relatively basic functional group (such as amino or guanidyl), an acid addition salt can be obtained by contacting the free form of the compound with a sufficient amount of acid, either in a pure solution or a suitable inert solvent. Non-limiting examples of pharmaceutically acceptable acid addition salts include, but are not limited to, inorganic acid salts (e.g., hydrochloride, hydrobromide, hydroiodide, nitrate, carbonate, bicarbonate, phosphate , monohydrogen phosphate, dihydrogen phosphate, phosphite, sulfate, bisulfate salts, *etc.*)*,* organic acid salts (such as acetate, propionate, isobutyrate, malonate, succinate, suberate, maleate, fumarate, citrate, tartrate, lactate, mandelate, benzoate, phthalate, mesylate, besylate, p-tosylate glucuronate salts, *etc.*) and amino acid salts (such as arginine salt, *etc.*)*.* For specific forms of pharmaceutically acceptable salts, also see Berge et al., "Pharmaceutical Salts", Journal of Pharmaceutical Science, 1977, 66: 1-19.

In the present disclosure, the term "metabolic disorder" can refer to disorders of glucose metabolism, such as diabetes, diabetes complications, obesity and obesity complications. Since the association between obesity, diabetes and blood glucose metabolism is well known, these conditions can, but need not be, separate or mutually exclusive. In some embodiments, diabetes or diabetes complications include insulin resistance, glucose intolerance, elevated fasting blood glucose, prediabetes, type I diabetes, type II diabetes, gestational diabetes hypertension, dyslipidemia, or a combination thereof. In some embodiments, obesity complications include obesity-associated inflammation, obesity-associated gallbladder diseases, or obesity-induced sleep apnea, or may be selected from the following related conditions: atherogenic dyslipidemia, dyslipidemia, increased blood pressure, hypertension, prothrombotic state and proinflammatory state, or a combination thereof.

The pharmaceutical composition of the present disclosure has excellent dual GIPR and GLP-1R agonist activity, is capable of effectively reducing blood glucose and controlling body weight gain in type II diabetes model mice, is useful in the prevention and/or treatment of metabolic disorders, and thus has good clinical application and pharmaceutical use.

### <Dual GLP-1 and GIP receptor agonists>

In the present disclosure, the dual GLP-1 and GIP receptor agonist refers to a substance that targets both the GLP-1 receptor and the GIP receptor and has activity of activating both GLP-1R and GIPR.

In some specific embodiments, the dual GLP-1 and GIP receptor agonist described in the present disclosure is a compound represented by formula I or a pharmaceutically acceptable salt, ester, solvate, optical isomer, tautomer, isotopically labeled compound or prodrug thereof:

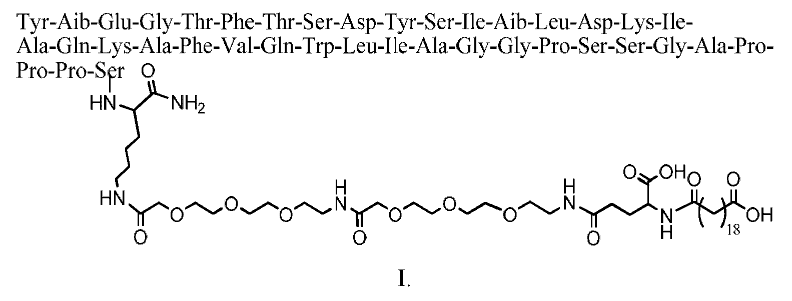

The content of the pharmaceutical active ingredient in the pharmaceutical composition is not particularly defined in the present disclosure, and may be determined by those skilled in the art according to actual needs.

In some embodiments, the concentration of the dual GLP-1 and GIP receptor agonist described in the present disclosure is 2-36 mg/mL; e.g. 2 mg/mL, 3 mg/mL, 4 mg/mL, 5 mg/mL, 6 mg/mL, 7 mg/mL, 8 mg/mL, 9 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL, 20 mg/mL, 21 mg/mL, 22 mg/mL, 23 mg/mL, 24 mg/mL, 25 mg/mL, 26 mg/mL, 27 mg/mL, 28 mg/mL, 29 mg/mL, 30 mg/mL, 31 mg/mL, 32 mg/mL, 33 mg/mL, 34 mg/mL, 35 mg/mL, 36 mg/mL, etc.; preferably 5-30 mg/mL.

### <Stabilizers>

In the present disclosure, the stabilizer is a substance used to stabilize the structure and biological activity of a polypeptide-based substance in a solution. In particular for a polypeptide-based preparation, screening a suitable stabilizer is a main means for preventing physical and chemical degradation of a polypeptide-based drug molecule in a liquid preparation. Choosing a suitable stabilizer can improve the stability of a polypeptide molecule by binding of the stabilizer to a hydrophobic portion of the polypeptide molecule, increasing the viscosity of the solution, and influencing a folding state of the polypeptide molecule.

The specific types of stabilizers are not particularly defined in the present disclosure, and may be determined by those skilled in the art according to actual needs.

In some embodiments, the stabilizer described in the present disclosure is selected from a polyol.

In some specific embodiments, the stabilizer described in the present disclosure is propylene glycol, mannitol, or glycerin.

Based on the results of the prescription screening test, in combination with the results of the stability test, propylene glycol is preferentially chosen as the stabilizer in the present disclosure, which is capable of effectively maintaining the structure and biological activity of the main drug, while also stabilizing the pH of the pharmaceutical composition during storage.

The usage amount of the above stabilizers is not particularly defined in the present disclosure, and may be determined by those skilled in the art according to actual needs.

In some embodiments, the concentration of the stabilizer described in the present disclosure is 10-20 mg/mL; e.g., 10 mg/mL, 11 mg/mL, 12 mg/mL, 13 mg/mL, 14 mg/mL, 15 mg/mL, 16 mg/mL, 17 mg/mL, 18 mg/mL, 19 mg/mL, 20 mg/mL, etc.; preferably 14 mg/mL.

It is worth mentioning that in polypeptide-based preparations, sodium chloride is believed to play a role in maintaining electrolyte balance and stabilizing polypeptide structures, and it is present as a stabilizer in a variety of existing polypeptide-based preparations. However, when the inventors of the present disclosure used sodium chloride instead of propylene glycol (e.g., 2 mL of a pharmaceutical composition comprised 40.0 mg of the compound of formula I, 3.58 mg of disodium hydrogen phosphate dodecahydrate, 16.4 mg of sodium chloride, with the pH adjusted to 7 using a pH regulator, and the balance made up with water for injection), the solubility of the compound of formula I was only 0.15 mg/mL, which was far from meeting the requirement. However, when propylene glycol was used as a stabilizer (e.g., 1 mL of a pharmaceutical composition comprised 20.0 mg of the compound of formula I, 3.58 mg of disodium hydrogen phosphate dodecahydrate, 14 mg of propylene glycol, with the pH adjusted to 7 using a pH regulator, and the balance made up with water for injection), the solubility of the compound of formula I can reach 30 mg/mL or more, meeting the solubility requirement of the main drug. Accordingly, in some preferred embodiments of the present disclosure, the pharmaceutical composition does not comprise sodium chloride.

### <pH and pH regulators>

The pH has a greater effect on the stability of the dual GLP-1 and GIP receptor agonist as a polypeptide-based drug, while it also affects the dissolution of the main drug during the formulation of the pharmaceutical composition. An environment of leaning acidity is detrimental to the dissolution of the dual GLP-1 and GIP receptor agonist provided in the present disclosure, and as the pH rises during product storage, the purity decreases rapidly, indicating the condition of leaning alkalinity is not conducive to the product stability.

In some embodiments, the pH of the pharmaceutical composition of the present disclosure is 6.5-8.5; e.g. 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, etc.; preferably 7.0-8.4; more preferably 7.2-8.2; and further preferably 7.2-7.6.

A reasonable pH range not only ensures the purity and stability of the pharmaceutical composition of the present disclosure during storage, but also allows for the compatibility of pH fluctuation during drug storage, thereby ensuring the stability of the pharmaceutical composition from production through use.

In some embodiments, a pH regulator is used in the present disclosure to adjust the pH of the pharmaceutical composition.

In some specific embodiment, the pH regulator described in the present disclosure is hydrochloric acid and/or sodium hydroxide. Hydrochloric acid is a clear, colorless, and fuming hydrochloric acid solution with a pungent odour that is often used as an acidifying agent in pharmaceutical preparations. Sodium hydroxide is a molten white dry particle, block, rod or flake, that is generally considered non-toxic at low concentrations, and widely applied in pharmaceutical preparations to adjust the pH of solutions. In some more specific embodiment, the pH regulator described in the present disclosure may be a hydrochloric acid solution at a concentration and/or a sodium hydroxide solution at a concentration.

The concentrations of the above pH regulators are not defined in the present disclosure, and may be adjusted by those skilled in the art according to actual production needs. For example, a lower concentration of a pH regulator may be selected, e.g. a pH regulator at a concentration below 3 M, or a higher concentration of a pH regulator may be selected, e.g. a pH regulator at a concentration above 3 M, which should have no adverse effect on pharmaceutical active ingredients. In the present disclosure, preferably 0.1 M hydrochloric acid and/or 0.1 M sodium hydroxide solutions are used.

### <Buffer salts>

In the present disclosure, a buffer salt refer to a substance used to stabilize the pH of a solution. In particular for a polypeptide-based preparation, screening a suitable buffer salt system is an important means for preventing physical and chemical degradation of a polypeptide-based drug molecule in a liquid preparation. Choosing a suitable buffer salt system can improve the stability of the polypeptide by keeping the pH of the polypeptide solution stable and influencing the electrostatic effect.

The specific types of buffer salts are not particularly defined in the present disclosure, and may be determined by those skilled in the art according to actual needs.

In some embodiments, the buffer salt described in the present disclosure is selected from a hydrogen phosphate, a hydrogen phosphate hydrate, a citrate, or a citrate hydrate.

In some specific embodiments, the buffer salt described in the present disclosure is disodium hydrogen phosphate dodecahydrate or sodium citrate dihydrate.

In some preferred embodiments, the buffer salt described in the present disclosure is disodium hydrogen phosphate dodecahydrate. It not only helps to stabilize the pH of the pharmaceutical composition of the present disclosure during storage, but also helps to stabilize the purity of the pharmaceutical composition during storage.

Based on the results of the prescription screening test, in combination with the stability test data, disodium hydrogen phosphate dodecahydrate is preferentially chosen as the buffer salt in the present disclosure, which has stable chemical properties, does not affect the quality and stability of the main drug, and well maintains stable pH of the preparation.

The usage mount of the above buffer salts is not particularly defined in the present disclosure, and may be determined by those skilled in the art according to actual needs.

In some embodiments, the concentration of the buffer salt described in the present disclosure is 7-23 mM; e.g., 7 mM, 8 mM, 9 mM, 10 mM, 11 mM, 12 mM, 13 mM, 14 mM, 15 mM, 16 mM, 17 mM, 18 mM, 19 mM, 20 mM, 21 mM, 22 mM, 23 mM, etc.; preferably 7 to 20 mM; and more preferably 7-15 mM.

Based on the results of the prescription screening test, taking into account the degree of the risk of the pharmaceutical composition as an injection preparation and the like in subsequent clinical application, a low concentration of buffer salt, such as a concentration of 10 mM, is preferentially chosen in the present disclosure.

### <Antimicrobial agents>

The dual GLP-1 and GIP receptor agonist described in the present disclosure is a linear peptide of 40 amino acids, and the preparation has a pH initially set between 7.2-8.2 and is extremely susceptible to degradation by microorganisms; therefore, the addition of an antimicrobial agent (such as phenol) to a prescription is generally considered to improve the product stability.

Furthermore, since a polypeptide-based drug is extremely susceptible to degradation by microorganisms, the addition of an antimicrobial agent to a polypeptide-based hypoglycemic drug is a conventional technical means in the art, for example, the commercially available hypoglycemic drug product, semaglutide (OZEMPIC) has 8.25 mg of phenol added as an antimicrobial agent; exenatide has 2.64 mg m-cresol added as an antimicrobial agent; liraglutide (VICTOZA) has 16.5 mg phenol added as an antimicrobial agent, and lixisenatide (ADLYXIN) has 8.1 mg m-cresol added as an antimicrobial agent.

However, the inventors of the present disclosure have found through long-term exploration and research that the safety of clinical medication can also be ensured without the addition of antimicrobial agents and risk factors of the pharmaceutical composition during application can be further reduced.

In the absence of an antimicrobial agent in the composition, it is more favorable to control or reduce the risk of the pharmaceutical preparation in clinical use, and reduce the production cost of the pharmaceutical composition to a certain extent, which is more favorable to large-scale production and application of drugs.

### <Pharmaceutical compositions>

The pharmaceutical composition of the present disclosure comprises a dual GLP-1 and GIP receptor agonist, a stabilizer, and a buffer salt.

In some embodiments, the pharmaceutical composition described in the present disclosure also comprises a pH regulator and a solvent.

In some specific embodiments, the pharmaceutical composition described herein consists of a dual GLP-1 and GIP receptor agonist, a stabilizer, a buffer salt, a pH regulator and a solvent.

The pharmaceutical composition provided in the present disclosure exhibits a good activation effect on GLP-1R and GIPR in vivo. The description of each ingredient in the pharmaceutical composition is as described above.

In some embodiments, the pharmaceutical composition of the present disclosure is a liquid preparation.

In some specific embodiments, the pharmaceutical composition described in the present disclosure is an injection.

In some more specific embodiments, the solvent used in the pharmaceutical composition of the present disclosure when present as a liquid preparation is water, preferably purified water or water for injection.

The present disclosure also provides a method for preparing said pharmaceutical composition, comprising the following steps: formulating the stabilizer, the buffer salt and the dual GLP-1 and GIP receptor agonist into a solution, and adjusting the pH of the solution to a target value to obtain the pharmaceutical composition.

In some specific embodiments, the method for preparing said pharmaceutical composition comprises the following steps: dissolving the stabilizer and the buffer salt in a solvent, adding the dual GLP-1 and GIP receptor agonist, adding the pH regulator to make the dual GLP-1 and GIP receptor agonist dissolved completely, and adjusting the pH of the solution to a target value using the pH regulator to obtain the pharmaceutical composition.

In some more specific embodiments, the method for preparing said pharmaceutical composition comprises the following steps: dissolving a prescribed amount of disodium hydrogen phosphate dodecahydrate and propylene glycol in purified water or water for injection, adding a prescribed amount of the dual GLP-1 and GIP receptor agonist, and adding sodium hydroxide to make the dual GLP-1 and GIP receptor agonist dissolved completely, adjusting the pH of the solution to 6.5-8.5 using hydrochloric acid and/or sodium hydroxide, and using purified water or water for injection for making up to a volume to obtain the pharmaceutical composition. In the method, the addition of sodium hydroxide is intended to raise the pH of the solution appropriately and thus is more beneficial for facilitating the dissolution of the dual GLP-1 and GIP receptor agonist.

In the present disclosure, the routes of administration can be varied or adjusted in any suitable manner to meet the needs of the drug nature, the convenience of the patient and medical personnel, and other relevant factors.

### <Pharmaceutical usages of pharmaceutical compositions>

The pharmaceutical composition of the present disclosure may be used for preventing and/or treating metabolic disorder related diseases; preferably, the metabolic disorder related diseases are diabetes, diabetes complications, obesity, or obesity complications.

The pharmaceutical composition of the present disclosure may be further used in the preparation of a drug for preventing and/or treating metabolic disorder related diseases; preferably, the metabolic disorder related diseases are diabetes, diabetes complications, obesity, or obesity complications.

The present disclosure also provides a method for preventing and/or treating metabolic disorder related diseases, comprising administering to a subject a prophylactically and/or therapeutically effective amount of the pharmaceutical composition according to the present disclosure; preferably, the metabolic disorder related diseases are diabetes, diabetes complications, obesity, or obesity complications.

### Examples

In order to make the objects and technical solutions of the present disclosure clearer, embodiments of the present disclosure are described in detail below in connection with examples. It will be appreciated by those skilled in the art that the following examples are merely illustrative of the present disclosure and should not be construed as limiting the scope of the present disclosure.

The reagents or instruments used in the examples are all conventional products that are commercially available. Examples, in which no specific conditions are specified, are carried out according to conventional conditions or conditions recommended by the manufacturers.

### Example 1: Prescription screening

In light of characteristics of this product as an injection dosage form, screening will now be conducted on the concentrations and types of buffer salts, the types of stabilizers, and the pH ranges of drug solutions, in the prescription. The compound of formula I used in the prescription study was self-made by the applicant, and the rest of materials used were commercially available.

### (1) Screening of types of stabilizers

A stabilizer can improve the stability of a polypeptide preparation by binding of the stabilizer to a hydrophobic portion of the polypeptide molecule, increasing the viscosity of the solution, and influencing a folding state of the polypeptide molecule. In order to screen for suitable stabilizers, preparations were prepared using propylene glycol, mannitol, and glycerin, respectively. The prepared samples were placed under conditions at 40°C and 2-8°C for high temperature and long-term investigation for stability. Samples were taken at different time points to examine the characteristics, pH and purity, and the effect of different stabilizers on the quality of the drug solution was investigated. The prescription information is shown in Table 1 and the results are shown in Table 2.

**Table 1 Prescription information**

| Composition of prescriptions | Prescription 1 | Prescription 2 | Prescription 3 |
|---|---|---|---|
| Compound of formula I | 400 mg | 400 mg | 400 mg |
| Disodium hydrogen phosphate dodecahydrate | 71.6 mg | 71.6 mg | 71.6 mg |
| Propylene glycol (injection grade) | 280 mg | NA | NA |
| Mannitol | NA | 280 mg | NA |
| Glycerin | NA | NA | 280 mg |
| 0.1 M hydrochloric acid/0.1 M sodium hydroxide solution | pH adjusted to 8.0 | pH adjusted to 8.0 | pH adjusted to 8.0 |
| Purified water | Added to 20 ml | Added to 20 ml | Added to 20 ml |

| | | | |
|---|---|---|---|
| Notes: "NA" denotes no addition. | | | |

**Table 2 Results of screening of types of stabilizers**

| Stabilizers | Stability conditions | Time | Characteristics | pH | Purity (%) | Maximum single impurity (%) |
|---|---|---|---|---|---|---|
| Propylene glycol (injection grade) | 40°C | 0 day | Colorless clear liquid | 8.4 | 93.41 | 5.35 |
| | | 10 days | Colorless clear liquid | 8.2 | 87.99 | 5.67 |
| | 2-8°C | 0 day | Colorless clear liquid | 8.4 | 93.41 | 5.35 |
| | | 1 month | Colorless clear liquid | 8.1 | 93.39 | 5.10 |
| Mannitol | 40°C | 0 day | Colorless clear liquid | 8.3 | 93.80 | 5.03 |
| | | 10 days | Colorless clear liquid | 7.5 | 89.29 | 5.08 |
| | 2-8°C | 0 day | Colorless clear liquid | 8.3 | 93.80 | 5.03 |
| | | 1 month | Colorless clear liquid | 8.2 | 93.27 | 5.09 |
| Glycerin | 40°C | 0 day | Colorless clear liquid | 8.4 | 93.20 | 5.37 |
| | | 10 days | Colorless clear liquid | 7.7 | 88.60 | 5.11 |
| | 2-8°C | 0 day | Colorless clear liquid | 8.4 | 93.20 | 5.37 |
| | | 1 month | Colorless clear liquid | 8.2 | 93.50 | 5.05 |

The samples using the stabilizers were all relatively stable under the condition of 2-8°C, and after 1 month of placement, there was no obvious change in the characteristics, pH, purity and the maximum single impurity of the samples. After the samples were placed at a high temperature of 40°C for 10 days, they had reduced purity, and there was no obvious change in the characteristics and the maximum single impurity. However, it was surprisingly found that the prescription with propylene glycol as the stabilizer, under conditions of the high-temperature accelerated stability experiment, had a pH change of less than 3% and can maintain pH stability, indicating that the prescription with propylene glycol as the stabilizer had achieved an unexpected technical effect in maintaining pH stability. In contrast, the prescription samples with mannitol and glycerin as stabilizers, under conditions of the high-temperature accelerated stability experiment, had a relatively great pH change with the pH drop of 0.7-0.8, and there was an obvious downward trend in pH. Therefore, the stabilizer for the product is preferably propylene glycol.

### (2) Screening of pH ranges

The pH is an important quality property of a polypeptide-based pharmaceutical preparation, and directly affects the solubility, stability and aggregation of the polypeptide molecule. The present disclosure investigated the pH range of 5.0-8.4, and focused on the dissolution of the drug substance and the product stability. The prescription information is shown in Table 3 and the results are shown in Table 4.

**Table 3 Prescription information**

| Prescription information | Prescription 4 | Prescription 5 | Prescription 6 | Prescription 7 | Prescription 8 |
|---|---|---|---|---|---|
| Target pH | 5.0 | 6.0 | 7.4 | 8.0 | 8.4 |
| Compound of formula I | 200 mg | 200 mg | 200 mg | 200 mg | 200 mg |
| Sodium citrate dihydrate | 58.8 mg | 58.8 mg | 58.8 mg | 58.8 mg | 58.8 mg |
| Propylene glycol (injection grade) | 140 mg | 140 mg | 140 mg | 140 mg | 140 mg |
| Phenol | 55 mg | 55 mg | 55 mg | 55 mg | 55 mg |
| 0.1 M hydrochloric acid/0.1 M sodium hydroxide solution | Q.S. | Q.S. | Q.S. | Q.S. | Q.S. |
| Purified water | Made up to 10 ml | Made up to 10 ml | Made up to 10 ml | Made up to 10 ml | Made up to 10 ml |

**Table 4 Results of preliminary confirmation for pH ranges of preparations**

| Target pH | Conditions | Time of maintaining stability | Characteristics | pH | Purity (%) |
|---|---|---|---|---|---|
| 5.0 | The drug substance was not completely dissolved, and there was a colloidal and transparent substance in the solution | | | | |
| 6.0 | The drug substance was not completely dissolved, and there was a colloidal and transparent substance in the solution | | | | |
| 7.4 | 40°C | 0 day | Colorless clear liquid | 7.5 | 95.39 |
| | | 10 days | Colorless clear liquid | 7.5 | 93.09 |
| | | 30 days | Colorless clear liquid | 7.5 | 89.05 |
| | 25°C | 0 day | Colorless clear liquid | 7.5 | 95.39 |
| | | 1 month | Colorless clear liquid | 7.5 | 94.59 |
| | | 3 months | Colorless clear liquid | 7.5 | 92.35 |
| 8.0 | 40°C | 0 day | Colorless clear liquid | 8.0 | 97.03 |
| | | 10 days | Colorless clear liquid | 8.0 | 93.66 |
| | | 30 days | Colorless clear liquid | 7.9 | 87.68 |
| | 25°C | 0 day | Colorless clear liquid | 8.0 | 97.03 |
| | | 1 month | Colorless clear liquid | 8.0 | 95.96 |
| | | 3 months | Colorless clear liquid | 8.0 | 91.03 |
| 8.4 | 40°C | 0 day | Colorless clear liquid | 8.4 | 96.75 |
| | | 10 days | Colorless clear liquid | 8.4 | 91.07 |
| | | 30 days | Colorless clear liquid | 8.4 | 81.63 |
| | 25°C | 0 day | Colorless clear liquid | 8.4 | 96.75 |
| | | 1 month | Colorless clear liquid | 8.4 | 94.90 |
| | | 3 months | Colorless clear liquid | 8.5 | 87.00 |

The results showed that the solubility of the drug substance at a pH of 5.0-6.0 can not meet the druggability requirement of the product, and the higher the pH was, the more conducive it was to the dissolution of the drug substance. In the target pH range of 7.4-8.4, the higher the pH was, the more the purity of the sample decreased under high temperature and accelerated conditions, i.e., the condition of leaning alkalinity was not conducive to the product stability. However, in terms of the degree of degradation, the reduction in product purity was still acceptable at the target pH of 8.4. There was not obvious change in other investigated items (such as characteristics and pH) among samples.

### (3) Screening of buffer salt concentrations

In general, pH and ionic strengths have a great influence on the stability and solubility of a polypeptide, improving the stability and safety of a polypeptide-based drug while ensuring its solubility has always been a technical problem that polypeptide pharmaceutical preparations need to solve, and a suitable buffer salt concentration is conducive to solving this technical problem. Therefore, in order to screen for suitable buffer salt concentrations, sodium citrate dihydrate at concentrations of 10 mM, 15 mM, and 20 mM was used for formulation of preparations respectively, and prepared samples were placed under conditions at 40°C and 25°C respectively for high temperature and long-term investigation for relevant indicators. The specific prescription information is shown in Table 5 and the results are shown in Table 6.

**Table 5 Prescription information**

| Prescription information | Prescription 9 | Prescription 10 | Prescription 11 |
|---|---|---|---|
| Target pH | 8.0 | 8.0 | 8.0 |
| Compound of formula I | 200 mg | 200 mg | 200 mg |
| Sodium citrate dihydrate | 29.4 mg (10 mM) | 44.1 mg (15 mM) | 58.8 mg (20 mM) |
| Propylene glycol (injection grade) | 140 mg | 140 mg | 140 mg |
| 0.1 M hydrochloric acid/0.1 M sodium hydroxide solution | Q.S. | Q.S. | Q.S. |
| Purified water | Made up to 10 ml | Made up to 10 ml | Made up to 10 ml |

**Table 6 Screening results of buffer salt concentrations**

| Buffer salt concentrations | Condit ions | Time | Characteristics | pH | % purity of the compound of formula I | Contents of the compound of formula I (mg/ml) |
|---|---|---|---|---|---|---|
| 10 mM | 40°C | 0 day | Colorless clear liquid | 8.0 | 97.71% | 17.04 |
| | | 10 days | Colorless clear liquid | 8.0 | 94.14% | 16.18 |
| | | 30 days | Colorless clear liquid | 8.0 | 88.06% | 15.80 |
| | 25°C | 0 day | Colorless clear liquid | 8.0 | 97.71% | 17.04 |
| | | 1 month | Colorless clear liquid | 8.0 | 96.55% | 16.75 |
| 15 mM | 40°C | 0 day | Colorless clear liquid | 7.9 | 97.43% | 16.92 |
| | | 10 days | Colorless clear liquid | 7.9 | 94.18% | 15.98 |
| | | 30 days | Colorless clear liquid | 7.9 | 88.38% | 15.01 |
| | 25°C | 0 day | Colorless clear liquid | 7.9 | 97.43% | 16.92 |
| | | 1 month | Colorless clear liquid | 7.9 | 96.29% | 17.13 |
| 20 mM | 40°C | 0 day | Colorless clear liquid | 8.0 | 97.06% | 16.25 |
| | | 10 days | Colorless clear liquid | 8.0 | 93.57% | 15.54 |
| | | 30 days | Colorless clear liquid | 8.0 | 87.05% | 15.10 |
| | 25°C | 0 day | Colorless clear liquid | 8.0 | 97.06% | 16.25 |
| | | 1 month | Colorless clear liquid | 8.0 | 95.82% | 15.65 |

The results showed that when injection samples formulated at different buffer salt concentrations were placed under a condition of 25°C for 1 month, only the purity of the prescription with 10 mM buffer salt concentration was slightly better than that of the prescriptions with 15 mM and 20 mM buffer salt concentrations; there was no obvious difference in each of other investigated items; and the buffer salt concentrations of 10 mM-20 mM can meet the requirements. However, considering that the higher the usage amount of auxiliary materials in an injection, the higher the risk of clinical application of preparations, the lowest concentration, 10 mM, was chosen as the buffer salt concentration of the injection of the present disclosure, so that a greater guarantee of the safety in using the preparations was provided.

### (4) Screening of buffer salt systems

The choice of a buffer system is also essential for improving the stability of a preparation. In order to screen for a suitable buffer salt, sodium citrate dihydrate and disodium hydrogen phosphate dodecahydrate were used for the formulation of preparations respectively, and prepared samples were placed under conditions of 40°C and 2-8°C respectively for high temperature and long-term investigation for relevant indicators. The specific prescription information is shown in Table 7 and the results are shown in Table 8.

**Table 7 Prescription information**

| Composition of prescriptions | Prescription 12 | Prescription 13 |
|---|---|---|
| Compound of formula I | 400 mg | 400 mg |
| Sodium citrate dihydrate (10 mM) | 58.8 mg | NA |
| Disodium hydrogen phosphate dodecahydrate (10 mM) | NA | 71.6 mg |
| Propylene glycol (injection grade) | 280 mg | 280 mg |
| 0.1 M hydrochloric acid/0.1 M sodium hydroxide solution | pH adjusted to 8.0 | pH adjusted to 8.0 |
| Purified water | Added to 20 mL | Added to 20 mL |

| | | |
|---|---|---|
| Notes: "NA" denotes no addition. | | |

**Table 8 Screening results of buffer salt systems**

| Buffer salts | Stability conditions | Time | Characteristics | pH | Purity (%) | Maximum single impurity (%) |
|---|---|---|---|---|---|---|
| Disodium hydrogen phosphate dodecahydrate | High temperature of 40°C | 0 day | Colorless clear liquid | 8.2 | 93.34 | 5.56 |
| | | 10 days | Colorless clear liquid | 8.2 | 89.97 | 5.37 |
| | Long-term, 2-8°C | 0 day | Colorless clear liquid | 8.2 | 93.34 | 5.56 |
| | | 1 month | Colorless clear liquid | 8.1 | 94.13 | 4.79 |
| Sodium citrate dihydrate | High temperature of 40°C | 0 day | Colorless clear liquid | 8.3 | 93.34 | 5.56 |
| | | 10 days | Colorless clear liquid | 8.4 | 88.96 | 5.51 |
| | Long-term, 2-8°C | 0 day | Colorless clear liquid | 8.3 | 93.34 | 5.56 |
| | | 1 month | Colorless clear liquid | 8.2 | 94.10 | 4.78 |

The results showed that both disodium hydrogen phosphate dodecahydrate and sodium citrate can be used as the buffer salts of the injection of the present disclosure, and the prescription with phosphate had slightly better purity than the prescription with citrate after being placed at a high temperature condition of 40°C for 10 days; therefore, disodium hydrogen phosphate dodecahydrate can be preferably as the buffer salt.

### (5) Further confirmation for pH ranges

It can be seen from the results of preliminary confirmation for pH ranges that the neutral pH condition was beneficial for the product stability, and the closer to physiological pH the less irritating according to subcutaneous injection of the product as the route of administration; therefore, a test was designed to carry out an investigation with a narrower range around pH 7.4, utilizing disodium hydrogen phosphate dodecahydrate as the buffer salt, for further confirmation for the optimal pH of the product. The prescription information is shown in Table 9 and the results are shown in Table 10.

**Table 9 Prescription information**

| Prescription information | Prescription 14 | Prescription 15 | Prescription 16 |
|---|---|---|---|
| Target pH | 7.2 | 7.4 | 7.6 |
| Compound of formula I | 400 mg | 400 mg | 400 mg |
| Disodium hydrogen phosphate dodecahydrate | 71.6 mg | 71.6 mg | 71.6 mg |
| Propylene glycol (injection grade) | 280 mg | 280 mg | 280 mg |
| 0.1 M hydrochloric acid/0.1 M sodium hydroxide solution | Q.S. | Q. S. | Q.S. |
| Purified water | Made up to 20 ml | Made up to 20 ml | Made up to 20 ml |

**Table 10 Results of further confirmation for pH ranges of preparations**

| Target pH | Condit ions | Time | Characteristics | pH | Total impurity (%) | Maximum single impurity (%) | RRT1. 04 (%) | Multi mer (%) |
|---|---|---|---|---|---|---|---|---|
| 7.2 | 40°C | 0 day | Colorless clear liquid | 7.3 | 6.83 | 0.47 | 5.21 | 0.08 |
| | | 5 days | Colorless clear liquid | 6.8 | 7.25 | 0.45 | 5.23 | 0.20 |
| | | 10 days | Colorless clear liquid | 7.1 | 8.03 | 0.67 | 5.16 | 0.30 |
| | 2-8°C | 0 day | Colorless clear liquid | 7.3 | 6.83 | 0.47 | 5.21 | 0.08 |
| | | 1 month | Colorless clear liquid | 7.1 | 6.81 | 0.47 | 5.20 | 0.09 |
| 7.4 | 40°C | 0 day | Colorless clear liquid | 7.5 | 6.50 | 0.52 | 5.15 | 0.07 |
| | | 5 days | Colorless clear liquid | 7.5 | 7.14 | 0.40 | 5.19 | 0.20 |
| | | 10 days | Colorless clear liquid | 6.9 | 7.70 | 0.66 | 5.09 | 0.32 |
| | 2-8°C | 0 day | Colorless clear liquid | 7.5 | 6.50 | 0.52 | 5.15 | 0.07 |
| | | 1 month | Colorless clear liquid | 7.5 | 6.57 | 0.45 | 5.21 | 0.08 |
| 7.6 | 40°C | 0 day | Colorless clear liquid | 7.7 | 6.61 | 0.54 | 5.22 | 0.08 |
| | | 5 days | Colorless clear liquid | 7.7 | 7.30 | 0.42 | 5.16 | 0.25 |
| | | 10 days | Colorless clear liquid | 7.7 | 8.52 | 0.66 | 5.21 | 0.59 |
| | 2-8°C | 0 day | Colorless clear liquid | 7.7 | 6.61 | 0.54 | 5.22 | 0.08 |
| | | 1 month | Colorless clear liquid | 7.7 | 6.46 | 0.46 | 5.18 | 0.09 |

The results showed that at the pH of 7.2-7.6, after the samples were placed at a high temperature of 40°C for 10 days, there was a slight increase in total impurities and multimers, and no obvious change in characteristics, pH, maximum single impurity and RRT 1.04; after the samples were placed under the long-term condition for 1 month, there was no obvious change in each investigated item; and there was no significant difference among the samples at different pH. Moreover, the prescriptions of the present disclosure without addition of antimicrobial agents can still control impurities within a range of no obvious change, achieving unexpected technical effects.

In other examples of the present disclosure, the concentration of the active pharmaceutical ingredient (API) can also be 5 mg/mL, 10 mg/mL, 15 mg/mL, 25 mg/mL, and 30 mg/mL, and the concentrations of other auxiliary materials were not changed. It will be understood that those skilled in the art may also adjust the API concentration according to specific conditions of a patient, all of the adjustments will fall within the scope of protection of the present disclosure.

In the present disclosure, the purified water in the above prescriptions 1-16 may be replaced with water for injection, and there was no obvious difference between all the experimental result data obtained from prescriptions with the water for injection and all the experimental result data obtained from the prescriptions with above purified water.

### Example 2: Preparation of the dual GIP and GLP-1 receptor agonist pharmaceutical composition

The composition was prepared by taking prescription 15 as an example. 71.6 mg of disodium hydrogen phosphate dodecahydrate (a buffer salt) and 280 mg of propylene glycol (for injection) (a stabilizer) were dissolved in purified water, 400 mg of a compound of formula I was added, 0.1 M sodium hydroxide solution (a pH regulator) was added simultaneously, and stirred until the starting material was dissolved, and the pH was adjusted with 0.1 M hydrochloric acid or 0.1 M sodium hydroxide solution to the target pH of 7.4. Finally, the volume was made up to 20 mL with purified water. The remaining prescriptions were prepared with reference to this preparation method.

### Example 3: Detection of multimer impurities in the dual GLP-1 and GIP receptor agonist pharmaceutical composition

### 1. The multimer detection method

Study content: Interference of the blank solution and the blank auxiliary material to the multimer detection.

Expected requirements: The blank solution and the blank auxiliary material should not interfere with the multimer detection.

### Test process:

(1) Blank solution: Solvent.
(2) Blank auxiliary material solution: The blank auxiliary material was taken to obtain the blank auxiliary material solution.
(3) System suitability solution: 10 mg of the control compound of formula I was taken, precisely weighed, and placed in a vial, and 5 ml of solvent was added to dissolve the compound by ultrasonic dissolution. The solution was placed at 60°C for 4 hours.
(4) Test sample solution: The dual GLP-1 and GIP receptor agonist pharmaceutical composition (the injection of the compound of formula I) was taken to obtain the test sample solution.
20 µL of the system suitability solution and 2 µL of the blank solution, the blank auxiliary material solution and the test sample solution were precisely measured out and injected into a liquid chromatograph, and the chromatograms were recorded.

### Test results:

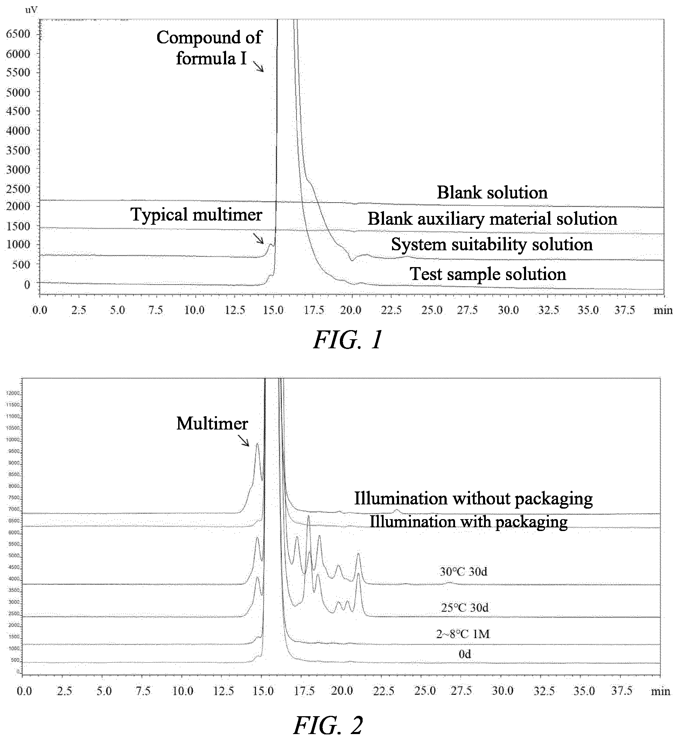

The blank solution and the blank auxiliary material did not interfere with the multimer detection. In the chromatogram of the system suitability solution, the peaks of the multimer and the compound of formula I appeared successively. The specific results were shown in Table 11 and FIG. 1.

**Table 11 Table of separation of the multimer and the product**

| Peak assignment | Positioning solution | System suitability solution | |
|---|---|---|---|
| | Retention time (min) | Retention time (min) | Relative retention time |
| Multimer | / | 14.798 | 0.95 |
| Compound of formula I | 15.567 | 15.587 | |

### 2. Detection of multimer impurities in the dual GLP-1 and GIP receptor agonist pharmaceutical composition

The compound of formula I was chemically synthesized polypeptide drug of which preparations may aggregate to form multimers during production and storage. The multimer was detected by SEC.

Prescription 15 was detected for the multimer, for investigating the multimer content of the prescription under a high temperature and illumination condition, the limit for the multimer was tentatively set at 2.0%, and specific data were shown in table 12 and FIG. 2.

**Table 12 The multimer detection results of the injection of the compound of formula I**

| Placement conditions | Placement time | Contents of multimers |
|---|---|---|
| Initial point | / | 0.09% |
| 2-8°C | 1 month | 0.09% |
| 25°C | 30 days | 0.63% |
| 30°C | 30 days | 0.71% |
| Illumination (2-8°C) With parkaging | With the total illuminance of not lower than 1.2 × 10⁶ lux·hr, and the near-ultraviolet lamp energy of not less than 200W·hr/m² | 0.07% |
| Illumination (2-8°C) Without packaging | | 1.3% |

It was surprisingly found that after the prescription sample was placed under the proposed storage condition (2-8°C) for 1 month, there was no change in the multimer content. The storage condition to be indicated in the package insert of this drug prescription after its expected marketing is 2-8°C, indicating that the prescriptions designed by the present disclosure solve the technical difficulty that polypeptide drugs are prone to polymerization, thereby ensuring the product stability after launching the drug on the market in the later stage. Moreover, even under harsher storage conditions, such as after storage at 25°C and 30°C for 30 days, respectively, the multimer increased slightly and the content thereof remained within the limit; and when the samples were placed at 2-8°C under illumination at a total illuminance of not less than 1.2 x 10⁶ lux·hr and the near-ultraviolet lamp energy of not less than 200W·hr/m², the multimer in unpackaged samples increased and the content thereof remained within the limit, and there was no significant change in the content of the multimer in packaged samples.

In summary, the ingredient of the product is stable under a suitable storage condition, with the multimer content within the limit.

## Claims

1. A pharmaceutical composition, **characterized by** comprising a dual GLP-1 and GIP receptor agonist, a stabilizer, and a buffer salt; wherein the dual GLP-1 and GIP receptor agonist is a compound represented by formula I or a pharmaceutically acceptable salt, ester, solvate, optical isomer, tautomer, isotopically labeled compound or prodrug thereof:

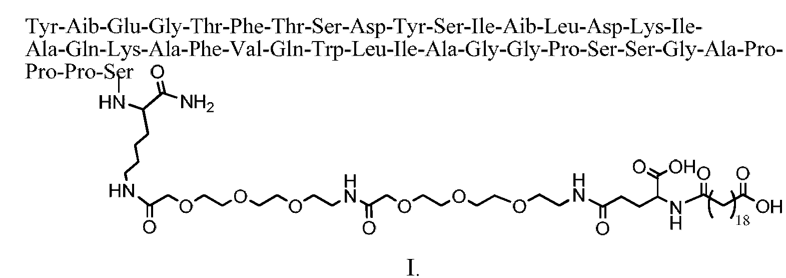

2. The pharmaceutical composition according to claim 1, **characterized in that** the pharmaceutical composition is a liquid preparation;
preferably, the pharmaceutical composition is an injection.

3. The pharmaceutical composition according to claim 2, **characterized in that** the concentration of the dual GLP-1 and GIP receptor agonist is 2-36 mg/mL.

4. The pharmaceutical composition according to any one of claims 1-3, **characterized in that** the stabilizer is a polyol;
preferably, the stabilizer is propylene glycol, mannitol or glycerin;
and more preferably, the stabilizer is propylene glycol.

5. The pharmaceutical composition according to any one of claims 2-4, **characterized in that** the concentration of the stabilizer in the pharmaceutical composition is 10-20 mg/mL.

6. The pharmaceutical composition according to any one of claims 1-5, **characterized in that** the buffer salt is selected from a hydrogen phosphate, a hydrogen phosphate hydrate, a citrate, or a citrate hydrate;
preferably, the buffer salt is disodium hydrogen phosphate dodecahydrate or sodium citrate dihydrate;
and more preferably, the buffer salt is disodium hydrogen phosphate dodecahydrate.

7. The pharmaceutical composition according to any one of claims 2-6, **characterized in that** the concentration of the buffer salt in the pharmaceutical composition is 7-23 mM.

8. The pharmaceutical composition according to any one of claims 1-7, **characterized in that** the pH of the pharmaceutical composition is 6.5-8.5;
preferably, the pH of the pharmaceutical composition is 7.0-8.4;
more preferably, the pH of the pharmaceutical composition is 7.2-8.2;
and further preferably, the pH of the pharmaceutical composition is 7.2-7.6.

9. The pharmaceutical composition according to any one of claims 1-8, **characterized in that** the pharmaceutical composition does not comprise an antimicrobial agent.

10. A pharmaceutical composition, **characterized by** comprising a dual GLP-1 and GIP receptor agonist represented by formula I, a stabilizer which is propylene glycol, and a buffer salt which is disodium hydrogen phosphate dodecahydrate, wherein the pH of the pharmaceutical composition is 7.0-8.4; moreover, in the pharmaceutical composition, the concentration of the dual GLP-1 and GIP receptor agonist represented by formula I is 5-30 mg/mL, the concentration of the propylene glycol is 10-20 mg/mL, and the concentration of the disodium hydrogen phosphate dodecahydrate is 7-23 mM; 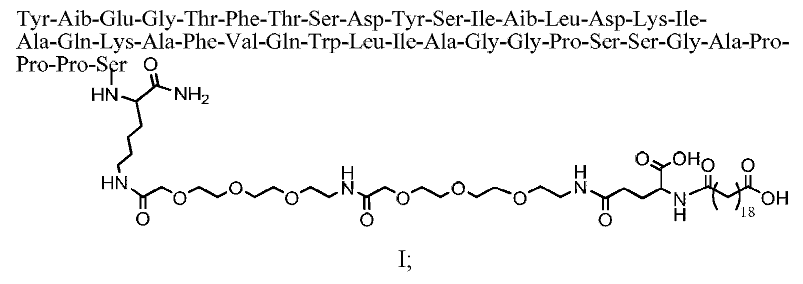 and the pharmaceutical composition is an injection.

11. The pharmaceutical composition according to claim 10, **characterized in that** the pH of the pharmaceutical composition is 7.2-8.2, and the concentration of the disodium hydrogen phosphate dodecahydrate in the pharmaceutical composition is 10-20 mM.

12. The pharmaceutical composition according to claim 10 or 11, **characterized in that** the pH of the pharmaceutical composition is 7.2-7.6; and in the pharmaceutical composition, the concentration of the dual GLP-1 and GIP receptor agonist represented by formula I is 5-25 mg/mL, the concentration of the propylene glycol is 14 mg/mL, and the concentration of the disodium hydrogen phosphate dodecahydrate is 10 mM.

13. The pharmaceutical composition according to any one of claims 10-12, **characterized in that** the pharmaceutical composition further comprises a pH regulator and water; and the pH regulator is hydrochloric acid and/or sodium hydroxide.

14. A method for preparing the pharmaceutical composition according to any one of claims 1-13, **characterized by** comprising the following steps: formulating the stabilizer, the buffer salt and the dual GLP-1 and GIP receptor agonist into a solution, and adjusting the pH of the solution to a target value to obtain the pharmaceutical composition.

15. Use of the pharmaceutical composition according to any one of claims 1-13 in the preparation of a drug for preventing and/or treating metabolic disorder related diseases, **characterized in that**
preferably, the metabolic disorder related diseases are diabetes, diabetes complications, obesity, or obesity complications.
